# EUROPEAN PATENT SPECIFICATION

(11) **EP 1 755 508 B1**
(45) Date of publication and mention of the grant of the patent: **13.10.2010**
(21) Application number: 05752538.8
(22) Date of filing: 16.06.2005
(51) Int. Cl.: A61F 11/14

(54) **CAP FOR USE AS HEARING PROTECTION AND METHOD FOR PRODUCING THE SAME**
KAPPE ZUR VERWENDUNG ALS HÖRSCHUTZ UND HERSTELLUNGSVERFAHREN DAFÜR
CASQUETTE DESTINEE A S'UTILISER POUR LA PROTECTION DE L'OUIE ET PROCEDE DE FABRICATION CORRESPONDANT

(30) Priority: 16.06.2004 SE 0401537
(43) Date of publication of application: 28.02.2007
(73) Proprietor: Sperian Hearing Protection, LLC, San Diego, CA 92154 (US)
(72) Inventor: HANSSON, Fredrik, S-254 58 Helsingborg (SE)
(74) Representative: Andersson, Mikael Per Robert
(86) International application number: PCT/SE2005/000924
(87) International publication number: WO 2005/122983

(56) References cited:
- US-A1- 5 718 002
- US-A1- 6 151 717

## Description

### Field of the Invention

The present invention relates to a cap intended for use as hearing protection and adapted to enclose the external ear of a user. The cap comprises a cap shell which is at least partly made of a substantially transparent material. Moreover the present invention relates to a method of producing a cap intended for use as hearing protection.

### Background Art

A common type of hearing protection comprises two caps which are connected by a headband. The headband is adapted to be placed over the head of the user and the two caps are adapted to be arranged one on each side of the user's head so as to enclose both external ears of the user. The cap in turn comprises a cap shell, which usually is made of plastic, a sealing ring, which is adapted to fit the head of the user, and some kind of sound-absorbing material arranged inside the cap. It is also common for a so-called bottom plate to be arranged which is located between the cap shell and the sealing ring and which keeps the sound-absorbing material in place inside the cap.

Hearing protectors are manufactured by specialised companies and are sold to other companies, which for instance have a production plant in which it is necessary to use hearing protectors, and to individuals. Other people who frequently use hearing protectors are, for instance, the staff at airports, motor races, roadworks etc. The companies buying hearing protectors often have various requirements concerning appearance or function. In many cases they want, for instance, the hearing protectors to carry the company's own logotype or some other type of information. Whether there are specific customer requirements or not, the company producing the hearing protector frequently wants to provide it with a picture of its own logotype.

To provide the hearing protectors with the desired pictures, the producers of hearing protectors use basically two alternatives which can be used either separately or in combination.

In a first alternative, special injection moulds are manufactured, in which cap shells with the raised logo-type of the customer company involved are produced. After that, optionally different colours are applied in order to provide a correct reproduction of the logotype intended. Since the tool cost is a considerable part of the cost of producing the hearing protectors, it is necessary for the customer company to purchase a large number of hearing protectors to make this alternative economically justifiable for the provision of customer-specific information.

In a second alternative, the logotype or some other specific information of the customer company is applied after injection moulding of the cap shell. Such subsequent application may consist of, for instance, printing or painting the logotype on the outside of the cap. The subsequent application may also involve gluing a printed decal to the outside of the cap. A drawback of this alternative is that the print or decal applied afterwards has a tendency to be worn off in connection with use and storing of the hearing protector in the user's locker. Above all the print or decal is worn when the user puts away the hearing protector or carries it hooked to his belt. Other occasions when the print or decal is worn is, for instance, in connection with the performance of certain welding, grinding and blasting operations and in the subsequent cleaning of the hearing protector. Exposure to the sun in outdoor work may also accelerate the reduction of the quality of the print or decal.

Thus, there is currently no satisfactory solution of how to provide, in a cost-effective and technically suitable manner, hearing protectors with information that is specific to the customer or the manufacturing company.

US-6,151,717 should be mentioned in the context. This publication discloses a hearing protector which is produced focusing on a very specific requirement. The publication discloses a cap provided with a window portion made of an optical material which lets through rays of light in such a manner that the human eye can see through the material to such an extent that an earmuff inserted into the ear can be discovered visually. The requirement in this case is that a foreman, safety representative or like person should be able to discover whether the user of the hearing protector provided with a window also uses earmuffs in the cases where this is stipulated by laws or other rules, without the user having to remove the outer hearing protector, which would mean that the user would have to interrupt his work and besides run the risk of hearing impairment. It should be noted in the context that this publication does not offer a solution to the above problem regarding how to provide, in a cost-effective and technically suitable manner, hearing protectors with information that is specific to the customer or the manufacturing company.

### Summary of the Invention

It is thus an object of the invention to provide a solution to the above-mentioned problem concerning how to provide, in a cost-effective and technically suitable manner, hearing protectors with information that is specific to the customer or the hearing protector manufacturing company.

According to the invention, this object is achieved by a cap of the type stated by way of introduction and given the characteristic features that at least one object or layer is arranged on an inner side of the cap shell which, in use of the cap, is arranged to face the user, and that said object or layer is visible through the cap shell.

In this way a cost-effective solution is achieved since the cap shells can be mass-produced without a customer-specific distinction between them and a technically suitable manner of applying the specific information is achieved since the information, for instance in the form of a printed logotype or decal, is protected by the cap shell and thus is not worn off in the manner as is a print or decal subsequently applied to the outside. By information is meant different types of information, such as text, numerals, figures, patterns, colour coding or some other type of visual distinction that is visible through the cap shell and that can be used to communicate a message to the user and to other people in the surroundings of the user. It should also be noted that the different types of information can also be combined with each other; advantageously it is possible to use, for instance, some kind of raised pattern from the moulding of the cap shell in combination with a partly translucent decal which contains text and picture and the colours of which are intensified by a coloured varnish which is applied to the inside of the cap shell and the decal so that the colour of the varnish is visible through the decal and the cap shell and respectively through the cap shell itself in areas outside the decal. The inventive cap can also be designed significantly more freely with regard to considerations of the design compared with conventional caps where any prints that are desirable in terms of design are quickly worn off. By considerations of the design are meant not only the aesthetical appearance but also, for instance, different types of colour coding of different sorts of hearing protector or colour coding that can be used to distinguish different groups of users, such as employees and visitors. By enabling mass-production of the cap shells without a customer-specific distinction, it will also be possible to offer more shapes or sizes of the cap for sale since within every such shape or size a larger volume is achieved compared with the case that the number of caps of a certain size must also be divided into a number of different customer-specific production series. Compared with the previously known caps as described above, there also occurs the option of arranging a luminous object, such as a diode, light bulb or the like or a chemically luminous object or the like, in the cap if, for some reason, it is important for the user to be actually seen. This could be the case, for instance, in outdoor work at night where it may be difficult to ensure satisfactory main lighting. Other occasions when it may be important to be seen involve roadworks, motor races or airports. With conventional caps, it would be necessary to make a compromise between providing the cap with an unwieldy encapsulation of the diode and other electronics on the outside of the cap and being forced to accept that the diode and the electronics are easily damaged in careless handling of the caps. Said objects or layers may also comprise reflectors or light-reflecting material in the cap. Since different types of reflectors can be arranged in the cap, it is possible to maintain large series of cap shells and nevertheless supply small series of special caps with reflectors of different colours.

Preferred embodiments of the inventive cap are defined by the dependent claims.

The cap shell including its see-through portion is advantageously made of a polymer material. In this manner, it is possible to use in a simple way conventional techniques to mould the cap shell to the desired shape.

According to a preferred embodiment, said object or layer carries information, such as text, picture, colour coding, luminous object or light-reflecting material. As mentioned above, it is possible to advantageously use the above-described invention to display different types of information, such as a company's name, logotype, classification of hearing protector or the like.

Advantageously, said object or layer is supplied to the cap shell on the side of the cap shell which, in use of the cap, is arranged to face the user. This results in a reduced risk of disadvantageous light refraction phenomena occurring since the object and the cap shell will fit tightly to each other. In addition, by arranging said object or layer on the cap shell, it is possible to construct the remaining parts of the cap in a conventional manner without having to take into great consideration, if at all, how said object or layer is arranged on the cap shell.

In a preferred embodiment, said object is attached to the cap shell by said object being at least partly moulded into the cap shell. In this way, the object will be securely attached. Moreover, since the object and the cap shell will fit tightly to each other, there will be a reduced risk that disadvantageous light refraction phenomena occur. Thus, the moulding into the cap shell helps to make the object distinctly visible through the cap shell.

Moreover, the cap advantageously comprises a pad of a sound-absorbing material, which is made up of one or more elements and is arranged in the cap so as to substantially cover the user's external ear. In this way, a good sound-absorbing function of the cap is obtained.

In a preferred embodiment, said object comprises at least one vibration-damping weight element of the cap, said weight element being arranged so as to at least partly block an audio mode of vibration that would have existed in the cap in the absence of said weight element. To block an audio mode of vibration means reducing or fully eliminating a resonance peak in the sound frequency spectrum. In this way, it is possible to drastically improve the damping capacity of the hearing protector for certain critical frequencies for which the cap otherwise runs the risk of having a relatively poor or even unsatisfactory damping. Moreover the transparent cap shell results in extra safety for the user since he can check that the vibration-damping weight elements are properly attached to the cap shell.

According to the invention, the above object is also achieved by a method of producing a cap intended for use as hearing protection, comprising the steps of providing a cap shell at least partly made of a substantially transparent material, providing at least one object or layer, arranging said at least one object or layer in the cap on an inner the side of the cap shell which, in use of the cap, is arranged to face the user, said object or layer being visible through the cap shell.

As mentioned above in connection with the cap, a cost-effective solution is achieved by this method since it is possible to mass-produce the cap shells without a customer-specific distinction between them and a technically suitable manner of applying the customer-specific information is achieved since this information, for instance in the form of a printed logotype or decal, is protected by the cap shell and thus is not worn off in the manner as is a print or decal subsequently applied to the outside. A number of additional advantages of the inventive method of producing the cap have been discussed in detail in connection with the inventive cap and, for the sake of clarity, it should be noted that these advantages are also achieved by the above-mentioned method of producing the cap.

The step of providing a cap shell advantageously comprises at least the steps of providing an openable and closable mould which in the closed state defines a cavity, supplying at least one substantially transparent polymer material in a mouldable state to the cavity defined by the mould to produce a cap shell, and removing from the mould the cap shell produced in the mould. In accordance with this method, caps can be produced in commercially effective and conventionally tested manufacturing processes at a commercially acceptable price and with satisfactory quality.

In a preferred embodiment, the method comprises the step of supplying, before the step of supplying a polymer material to the cavity, an object to the cavity, which object is arranged to be at least partly moulded into the cap shell. As mentioned above in connection with the cap, this promotes secure attachment of the object. Moreover, since the object and the cap shell will fit tightly to each other, there will be a reduced risk that unfavourable light refraction phenomena occur. The object can advantageously be customer-specific or in some other way specifically designed in the portion which merely is intended to be seen through the cap shell and be standardised in the portion which is intended to be held by the mould in connection with the moulding of the cap shell.

In another preferred embodiment, the above-mentioned method also comprises the step of supplying, after removing from the mould the cap shell produced in the mould, an object or layer on a side of the cap shell, which side is adapted to face the user, said object or layer being visible through the cap shell. In this way, information can easily be supplied, which is specific, for instance, for one customer although the customer buys only a limited number of hearing protectors. This information can be, for instance, a printed decal with the logotype of the company. It is also possible to use this method for applying a layer of coloured varnish, fluorescent paint or varnish, or the like, to provide colour coding of some kind. As mentioned above, this can also be used in combination with raised patterns, subsequent application of a decal or fixing an object by moulding.

Additional advantages and examples of advantageous use of the inventive cap will be evident from the detailed description.

### Brief Description of the Drawings

The accompanying schematic drawings illustrate currently preferred embodiments of the invention.
Fig. 1 is a cross-sectional view of a cap of a hearing protector according to a first embodiment.
Fig. 2 is a top plan view of the cap shown in Fig. 1 and illustrates the cap as it appears when viewed from the outside.
Fig. 3 is an exploded view corresponding to Fig. 1.
Fig. 4 is a perspective view of the cap shell in the first embodiment viewed from the outside.
Fig. 5 is a perspective view from the diametrically opposite direction in relation to Fig. 4 and shows the cap shell viewed from the inside.
Fig. 6 illustrates a hearing protector with a cap according to a second embodiment where the left cap is shown in the form of a cross-section.
Fig. 7 is a perspective view which shows an imaginary half of a cap shell corresponding to the cross-section in Fig. 6 of the complete cap.
Fig. 8 is a cross-sectional view of an injection mould intended for simultaneous production of two cap shells according to said second embodiment.

### Detailed Description of Preferred Embodiments

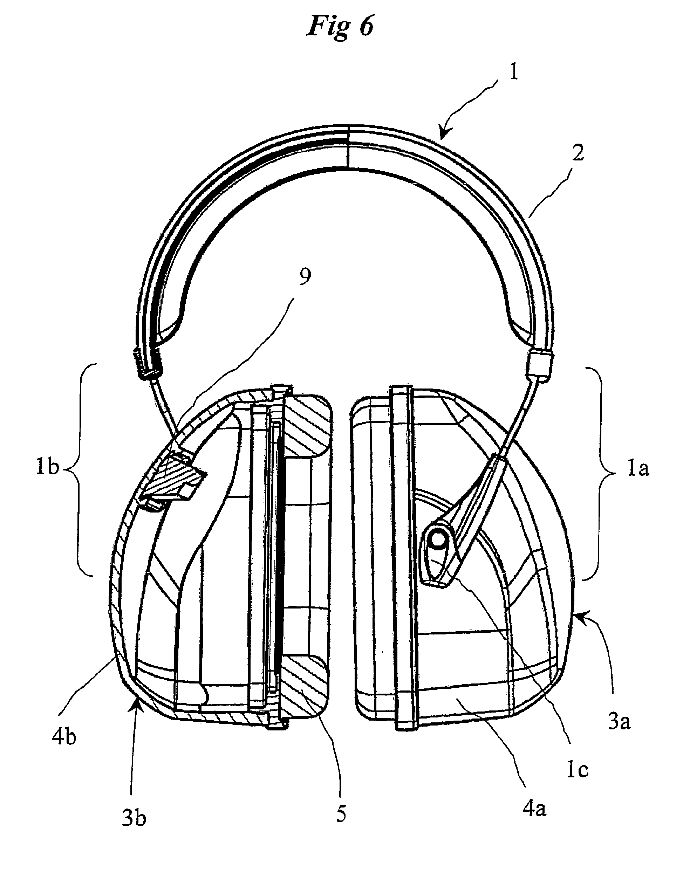

As is evident from Fig. 6, a hearing protector 1 comprises a headband 2 and two caps 3a-b. The headband 1 is intended to be placed over the user's head and the two caps 3a-b are intended to be placed one on each side of the user's head so as to enclose both external ears of the user.

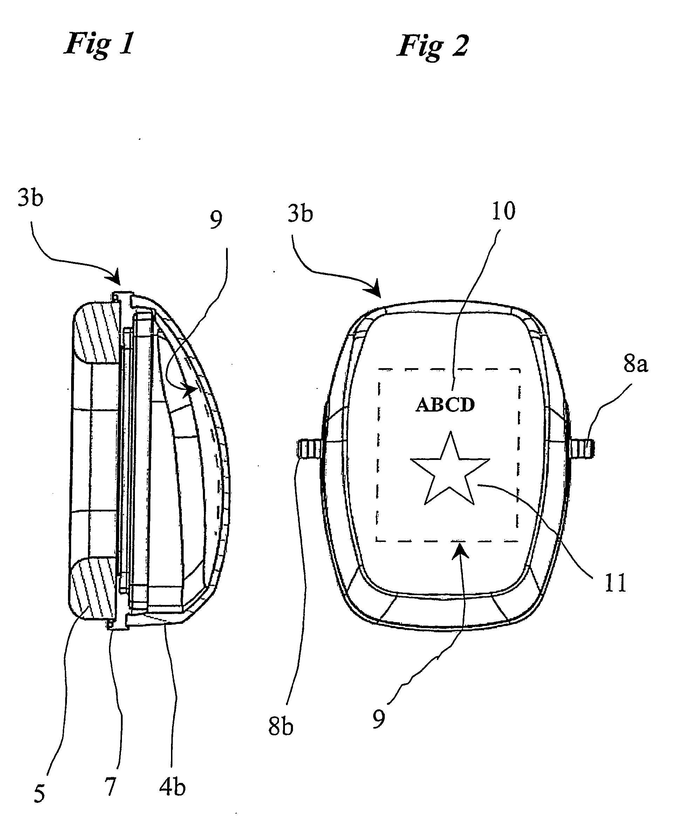

As is evident from Figs 1, 3 and 6, each cap 3a-b comprises in turn a cap shell 4, a sealing ring 5 and some kind of sound-absorbing material 6 arranged inside the cap 3a-b. Moreover, there is a bottom plate 7 which is positioned between the cap shell 4 and the sealing ring 5 and which keeps the sound-absorbing material 6 in place inside the cap 3a-b. The sealing ring 5 is adapted to fit the head of the user.

The headband 2 also has the function of pressing the caps 3a-b against the user's head with sufficient force to prevent air and sound from leaking in between the sealing ring 5 and the user's head.

The headband 2 allows the caps to be carried and is usually provided with two Y-shaped fork portions 1a, 1b. In each fork portion 1a-b, the two legs extend on opposite sides of the respective caps 3a-b. The caps 3a-b are in turn usually provided with two pins 8 a-b which extend from the respective caps 3a-b and engage in a recess 1c (Fig. 6 shows only the right fork portion 1a completely) in the corresponding leg of said fork portion 1a-b. As a result, the caps 4a-b will be pivotally carried by the headband 2. There are a number of commercially available techniques of attaching the caps 3a-b to the headband 1, and therefore it is not considered necessary to describe this part of the construction in more detail. The invention is not restricted to this type of connection between cap and headband but can be used with other types of connections. One example of another connection comprises a fork on each side of the headband, which engage a fixing point located in the centre of the cap. Such constructions are commonly used when it is desirable for the user to carry the headband in different positions, such as on top of his head, behind the nape of his neck or under his chin.

As is evident from Figs 2, 4 and 5, the cap shell 4 is made of a transparent material. The cap 3a-b is also arranged to carry at least one object 9 or layer on the side of cap shell 4 which, in use of the cap 3a-b, is arranged to face the user. Since the cap shell 4 is made of a transparent material, said object 9 or layer is visible through the cap shell 4.

Preferred materials for use in the production of said cap shells are clear amorphous polymers and crystalline PP (polypropylene). These materials provide a transparence or see-through effect which is satisfactory for this purpose. Especially preferred materials in this group are PP (polypropylene), ABS (acrylonitrile-butadiene-styrene copolymer), SBS (styrene-butadiene-based thermoplastic elastomer), MBS (methyl-methacrylate-butadiene-styrene), PC (polycarbonate resin), PET (polyethylene terephthalate) and PMMA (methyl-methacrylate resin). These can be used in injection moulding of the cap shell 4 more or less directly according to conventional methods.

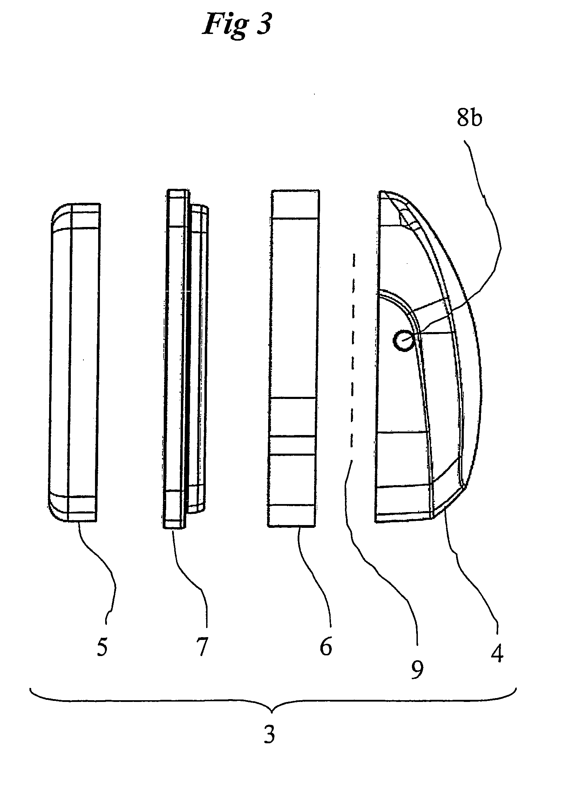

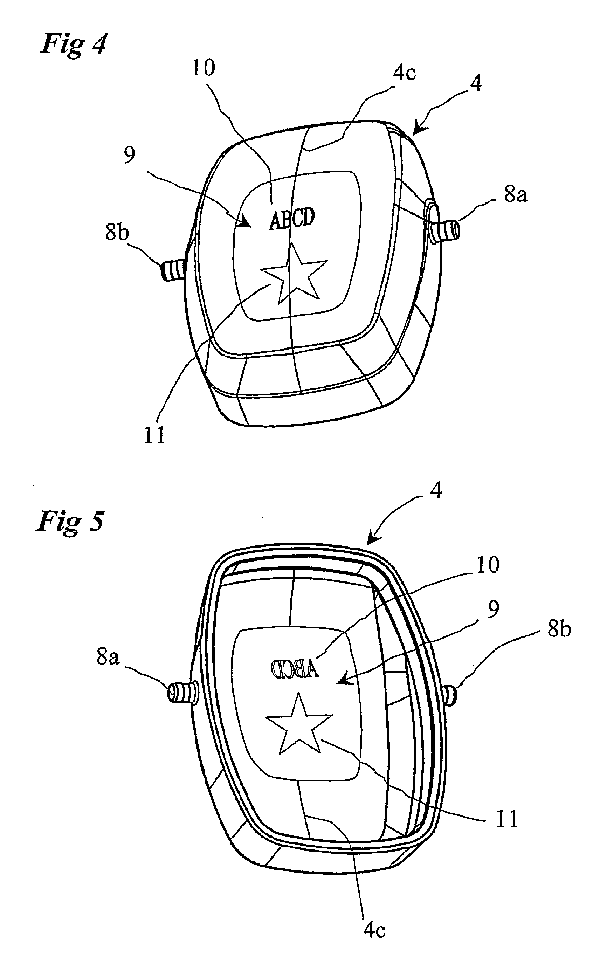

Figs 2-5 show an example of an object or layer in the form of a substantially rectangular decal 9 which carries text 10 and a picture 11. As seen from the exploded view in Fig. 3, the decal is arranged on the inside of the cap shell 4. This is also evident from Figs 4 and 5. The technique of drawing requires a transition of radius 4c in the centre of the cap shell 4. In Figs 4 and 5, this transition of radius 4c has been maintained since it illustrates the location of the decal 9 in relation to the cap shell 4. In the example shown in Fig. 5, the decal 9 is substantially opaque or non-transparent, which is made clear by the transition of radius 4c being visible from the inside of the cap shell 4 but being covered by the decal 9 so as not to be partially seen any longer in the centre of the cap shell 4. The decal 9 can also be made of a material which is more or less transparent. The text or picture can also be more or less transparent.

In a preferred embodiment, the decal 9 is substantially translucent and provided with a print in the form of a logotype or text. After applying the decal 9 to the inside of the cap shell 4, the inside of the cap shell 4 and, thus, the back of the decal 9 are coated with a coloured varnish or the like which is visible through the decal 9 and the cap shell 4. The back of the decal 9 refers to the side facing away from the cap shell 4. The front of the decal 9 refers to the side which faces the cap shell 4 and which is visible through the cap shell 4 also when non-see-through decals 9 are involved. By selecting an at least partly see-through print on the decal 9, it is possible to provide pictures that are highly true to life by using the fact that the applied coloured varnish is partly to be seen through the print and intensifies the colour thereof.

Also with non-see-through decals 9, it is in many cases desirable to apply a layer of coloured or transparent varnish to the inside of the cap shell since this improves the attachment of the decal 9 and protects it from being unintentionally affected, such as dried up, which may result in discoloration of the decal and print, and the adhesive, by which the decal is attached to the cap shell, losing its adhesivity.

It is also possible to use application of varnish in more than one layer. For example, the inside of the cap shell can be coated with a first layer of varnish and immediately after that the decal is applied before the layer of varnish has dried, thus using the layer of varnish as adhesive for the decal. In addition, this direct contact reduces the risk of undesirable light refraction phenomena. Optionally, the inside of the cap shell can then be coated with another layer that encapsulates the decal.

In the description above, the word varnish has been used for the substance that is applied as a layer. However, it should be noted that the layer can involve different types of paint or other substances that can be applied, for example, in the form of liquid or aerosol (such as spray paint). The layer may also consist of a second layer of plastic material which is applied, for instance, in a second injection-moulding step. The varnish, the paint or the plastic may carry optional types of colour pigments or other types of components which produce an effect that is visible through the cap shell. An example of such components is the type of pigment flakes that is used in what is referred to as metallic paint in car painting. It is also possible to use lacquer or paint that crackles in drying. Preferably, such a crackled lacquer or paint is coated with another layer of lacquer or paint on the inside of the cap shell.

The text and picture shown in Fig. 2 need not necessarily be applied by means of a printed decal but can also be printed directly on the inside of the cap shell 4 and preferably after that be coated with a lacquer on the inside of the cap shell 4 in the same way as described in connection with the decal 9.

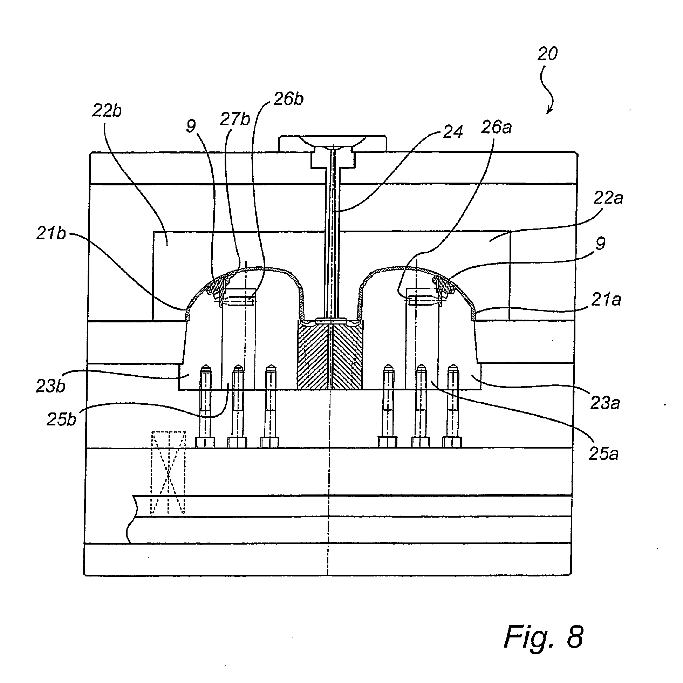

Fig. 6 illustrates another embodiment where the object 9 visible through the cap shell is partly moulded into the cap shell 4. This is provided, for example, by said object 9 being placed in the mould 20 which is used for injection moulding of the cap shell 4. Such a mould is shown in Fig. 8.

The mould 20 defines two cavities 21a-b, to which plastic is adapted to be supplied to form two cap shells 4a-b. The boundary surfaces of the cavities 21a-b are formed by outer moulds 21a-b and inner moulds 23a-b. The plastic is supplied through a runner 24 to the interior of the mould 20 and to the two cavities 21a-b. For the plastic to fill the cavities 21a-b completely, it is supplied in a heated, mouldable state under pressure. The selection of pressure and temperature depends on, inter alia, what type of polymer material is used and how complicated a shape the object to be injection moulded has. Moreover the shape and the necessary pressure will control if and, in that case, in what directions and along what parting lines it is suitable to divide the outer mould and or the inner mould into a plurality of parts. Injection moulding of polymer material is a commercially well-known technique, and since the above-mentioned decisions are generally known to a person skilled in the art, only one alternative will be described and only to a level of details that is necessary for the understanding of the invention.

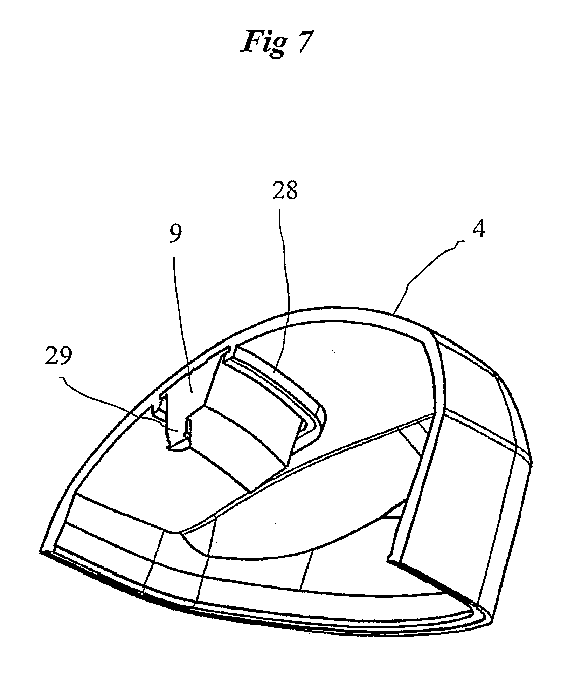

Fig. 8 shows how the inner mould 23a-b has been adjusted so as to each comprise a specially designed inner part 25a-b which in turn comprises a holder 26a-b. The holder 26a-b is arranged to hold an object 9 in the correct position in the cavity 21a-b so that the object 9 is partly moulded into the cap shell 4a-b as the plastic is supplied to the cavity 21a-b through said runner 24. The inner moulds 23a-b have further been formed with an annular recess 27a-b which extends around each of said objects 9. The recesses 27a-b will be filled with plastic and the cap shell 4 will thus be formed with an edge 28 extending around each of said objects 9 (see Fig. 7). The object 9 is provided with a pin 29 with which said holder 26a-b is arranged to engage to keep the object 9 in place in the cavities 21a-b.

The sound-absorbing material 9 is usually made of foam plastic, glass down, fibre cloth or textile forming a non-see-through pad.

Since the sound-absorbing material is porous and has a certain thickness, there are formed, for a ray of light directed through the sound-absorbing material, a large number of boundary layers between materials with different refractive indices or materials and air with different refractive indices. Therefore the sound-absorbing material will not be transparent whether the material from which the individual fibres are made or the material which is foamed is transparent or not. Only extremely thin materials would be translucent but they would not serve an acoustic purpose.

It should be noted that in this text the terms transparent and see-through have been used more or less as synonyms, but that in some cases sufficient transparency is provided if the cap shell has sufficient translucence. Such a case involves, for instance, a light-emitting diode arranged inside the cap shell. The degree of required transparence or translucence, of course, varies with the field of application, and the important thing is whether the object or layer located inside the cap shell is visible from the outside of the cap shell.

In another preferred embodiment, the object 9 shown in Fig. 7 comprises an encapsulated printed circuit card with a battery, a control circuit and one or more lamps or diodes facing the cap shell 4 so as to be visible through the cap shell 4. The cap shell 4 is advantageously made by injection moulding or the like and has there been provided with an edge 28 that forms a space in which said encapsulated printed circuit card is arranged to be secured by a tight fit or the like.

In yet another preferred embodiment, the object 9 comprises a reflector which is attached to the cap by an adhesive, by being screwed or riveted at certain points or by being clamped, for instance between the cap shell and an edge of the bottom plate, in mounting of the cap. The reflector 9 can have different sizes, shapes or colours, which in itself can be intended to communicate a quantity of information. The reflector can also be secured to the cap shell in the way shown in Fig. 7 by a subsequent tight fit or by being located in the mould in injection moulding of the cap shell.

In a further preferred embodiment, said object 9 of the caps 4a-b also has the function of serving as a vibration-damping weight element which is arranged so as to at least partly block an audio mode of vibration that would have existed in the cap in the absence of said weight element. According to the definition above, an audio mode of vibration is a resonance peak in a sound frequency spectrum, i.e. a state of the cap in which the cap oscillates or vibrates in resonance with an incoming sound wave. To block an audio mode of vibration implies as stated above to reduce or eliminate a resonance peak in the sound frequency spectrum.

In the embodiment shown in Fig. 7, the weight element 9 is arranged by being partly moulded into the cap shell 4. Especially at the higher frequencies, from about 1 kHz upwards, vibrations occur in precisely the cap shell 4 of the cap owing to noise from the environment. By arranging the weight element 9 in the cap shell 4, it is possible to prevent the cap shell 4 from exhibiting certain undesirable natural frequencies around which it could otherwise come into resonance with incoming noise.

The weight element 9 advantageously has a density that is higher than the density of the rest of the cap shell 4. As a result, effective resonance absorption is achieved with a relatively small volume of the weight element 9. The difference in density can be provided by the weight element 9 being made of a heavy material such as metal, preferably zinc or a zinc alloy, while the cap shell 4 is in a conventional way made of plastic. Zinc has like many other metals, such as iron, a significantly higher density than plastic (in the order of seven times higher). In addition, zinc is relatively easy to form to the desired shape. The forming of the weight element 9 can occur by, for instance, die casting, punching, forging, turning, milling, drilling or die stamping, thereby achieving complex shapes for continued production.

It should be noted that even if the object is used as a weight element 9, the above-mentioned advantages of the see-through cap shell has not been missed. The object can on the side facing the cap shell 4 (and thus being visible through the same) be provided with a print, a raised logotype or the like. Since the cap shell is see-through, the user can also by ocular inspection verify that the weight element is attached to the cap and, thus, reduce the risk of discomfort or impaired hearing.

## Claims

1. A cap intended for use as hearing protection (1) and adapted to enclose the external ear of a user, comprising a cap shell (4a-b) at least partly made of a substantially transparent material, **characterised in that** at least one object or layer (9) is arranged on an inner side of the cap shell (4a-b) which, in use of the cap (3a-b), is arranged to face the user, and that said object or layer (9) is visible through the cap shell (4a-b).

2. A cap as claimed in claim 1, in which the cap shell (4a-b) including its transparent portion is made of a polymer material.

3. A cap as claimed in claim 1 or 2, in which said object or layer carries information, such as text, picture, colour coding, luminous object or light-reflecting material.

4. A cap as claimed in any one of the preceding claims, in which said object (9) is attached to the cap shell (4a-b) by said object (9) being at least partly moulded into the cap shell (4a-b).

5. A cap as claimed in any one of the preceding claims, which further comprises a pad (6) of a sound-absorbing material, which is made up of one or more elements and is arranged in the cap (3a-b) so as to substantially cover the user's external ear.

6. A cap as claimed in any one of the preceding claims, in which said object comprises at least one vibration-damping weight element (9) of the cap (3a-b), said weight element being arranged so as to at least partly block an audio mode of vibration that would have existed in the cap (3a-b) in the absence of said weight element (9).

7. A method of producing a cap intended for use as hearing protection (1), comprising the steps of
providing a cap shell (4a-b) at least partly made of a substantially transparent material,
providing at least one object or layer (9) arranging said at least one object or layer (9) in the cap (3a-b) on an inner side of the cap shell (4a-b) which, in use of the cap (3a-b), is arranged to face the user, said object or layer (9) being visible through the cap shell (4a-b).

8. A method as claimed in claim 7, in which the step of providing a cap shell (4a-b) at least comprises the steps of
providing an openable and closable mould (20) which in the closed state defines a cavity (21a-b),
supplying at least one substantially transparent polymer material in a mouldable state to the cavity (21a-b) defined by the mould (20) to produce a cap shell (4a-b), and
removing from the mould (20) the cap shell (4a-b) produced in the mould.

9. A method as claimed in claim 8, which further comprises the step of supplying, before the step of supplying a polymer material to the cavity (21a-b), an object (9) to the cavity (21a-b), which object (9) is arranged to be at least partly moulded into the cap shell (4a-b).

10. A method as claimed in claim 8, which further comprises the step of supplying, after removing from the mould (20) the cap shell (4a-b) produced in the mould (20), an object or layer (9) to the cap shell (4a-b) on a side of the cap shell (4a-b), which side is adapted to face the user, said object or layer (9) being visible through the cap shell (4a-b).

## Patentansprüche

1. Kappe, die zur Verwendung als Hörschutz (1) bestimmt ist und dazu geeignet ist, das Außenrohr eines Benutzers einzuschließen, umfassend eine Kappenschale (4a-b), die zumindest teilweise aus einem im Wesentlichen durchsichtigen Material hergestellt ist,
**dadurch gekennzeichnet, dass** zumindest ein Objekt oder eine Schicht (9) auf einer Innenseite der Kappenschale (4a-b) angeordnet ist, die bei Verwendung der Kappe (3a-b) dem Benutzer zugekehrt, angeordnet ist, und dass das Objekt oder die Schicht (9) durch die Kappenschale (4a-b) sichtbar ist.

2. Kappe nach Anspruch 1, wobei die Kappenschale (4a-b) mit ihrem durchsichtigen Abschnitt aus einem Polymermaterial hergestellt ist.

3. Kappe nach einem der Anspruche 1 oder 2, wobei das Objekt oder die Schicht Information trägt, wie etwa Text, Bild, Farbkennzeichnung, Leuchtobjekt oder lichtreflektierendes Material.

4. Kappe nach einem der vorhergehenden Ansprüche, wobei das Objekt (9) **dadurch** an der Kappenschale (4a-b) angebracht ist, dass das Objekt (9) zumindest teilweise in die Kappenschale (4a-b) geformt ist.

5. Kappe nach einem der vorhergehenden Ansprüche, ferner umfassend ein Polster (6) aus einem schalldämpfenden Material, das aus einem oder mehr Elementen hergestellt ist und derart in der Kappe (3a-b) angeordnet ist, dass es das Außenohr des Benutzers im Wesentlichen abdeckt.

6. Kappe nach einem der vorhergehenden Ansprüche, wobei das Objekt zumindest ein schwingungsdämpfendes Gewichtselement (9) der Kappe (3a-b) umfasst, wobei das Gewichtselement derart angeordnet ist, dass es einen Schwingungstonmodus, der in Abwesenheit des Gewichtselements (9) index Kappe bestanden hätte, zumindest teilweise blockiert.

7. Verfahren zum Erzeugen einer Kappe, die zur Verwendung als Hörschutz (1) bestimmt ist, umfassend die Schritte des
Bereitstellens einer Kappenschale (4a-b), die zumindest teilweise aus im Wesentlichen durchsichtigem Material hergestellt ist,
Bereitstellens von zumindest einem Objekt oder einer Schicht (9),
Anordnens von zumindest einem Objekt oder einer Schicht (9) in der Kappe (3a-b) auf einer Innenseite der Kappenschale (4a-b), die bei Verwerdung der Kappe (3a-b) dem Benutzer zugekehrt angeordnet ist, wobei das Objekt oder die Schicht (9) durch die Kappenschale (4ab) sichtbar ist.

8. Verfahren nach Anspruch 7, wobei der Schritt des Bereitstellens einer Kappenschale (4a-b) zumindest die Schritte des
Bereitstellens einer Form (20), die geöffnet und geschlossen werden kann, und die im geschlossenen Zustand einen Hohlraum (21a-b) definiert,
Zuführens von zumindest einem, im Wesentlichen durchsichtigen Polymermaterial in einem formbaren Zustand in den Hohlraum (21a-b), der durch die Form (20) definiert ist, zum Erzeugen einer Kappenschale (4a-b), und
Entfernens der Kappenschale (4a-b), die in der Form erzeugt wurde, aus der Form (20) umfasst.

9. Verfahren nach Anspruch 8, das ferner den Schritt des Zuführens, vor dem Schritt des Zuführens eines Polymermaterials in den Hohlraum (21a-b), eines Objekts (9) in den Hohlraum (21a-b) umfasst, wobei das Objekt (9) dazu angeordnet ist, zumindest teilweise in die Kappenschale (4a-b) geformt zu werden.

10. Verfahren nach Anspruch 8, das ferner den Schritt des Zuführens, nach dem Entfernen der Kappenschale (4a-b), die in der Form (20) erzeugt wurde, aus der Form (20), eines Objekts oder einer Schicht (9) zu der Kappenschale (4a-b) auf einer Seite der Kappenschale (4a-b) umfasst, die dazu geeignet ist, dem Benutzer zugekehrt zu sein, wobei das Objekt oder die Schacht (9) durch die Kappenschale (4a-b) sichtbar ist.

## Revendications

1. Capuchon destiné à être utilisé comme protection auditive (1) et adaptée pour envelopper l'oreille externe d'un utilisateur, comprenant une coquille de capuchon (4a-b) au moins partiellement constituée d'un matériau substantiellement transparent,
**caractérisé en ce qu'**au moins un objet ou une couche (9) est prévue sur un côté intérieur de la coquille de capuchon (4a-b), qui est adapté pour être tourné vers l'utilisateur pendant l'utilisation du capuchon (3a-b), et **en ce que** ledit objet ou ladite couche (9) est visible à travers la coquille de capuchon (4a-b).

2. Capuchon selon la revendication 1, dans lequel la coquille de capuchon (4a-b), y compris sa partie transparente, est constituée d'une matière polymère.

3. Capuchon selon la revendication 1 ou 2, dans lequel ledit objet ou ladite couche porte des informations, telles que du texte, des images, des codes couleur, un objet lumineux ou un matériau réfléchissant.

4. Capuchon selon l'une quelconque des revendications précédentes, dans lequel ledit objet (9) est attaché à la coquille de capuchon (4a-b), ledit objet (9) étant au moins partiellement moulé dans la coquille de capuchon (4a-b).

5. Capuchon selon l'une quelconque des revendications précédentes, comprenant en outre un coussinet (6) constitué d'un matériau d'absorption sonore, composé d'un ou de plusieurs éléments et agencé dans le capuchon (3a-b), de manière à recouvrir substantiellement l'oreille externe de l'utilisateur.

6. Capuchon selon l'une quelconque des revendications précédentes, dans lequel ledit objet comprend au moins un poids amortisseur de vibrations (9) du capuchon (3a-b), ledit poids étant conçu de manière à bloquer au moins partiellement un mode de vibration audio qui aurait été présent dans le capuchon (3a-b) en l'absence dudit poids (9).

7. Méthode pour la production d'un capuchon destiné à être utilisé comme protection auditive (1), comprenant les étapes suivante
mise à disposition d'une coquille de capuchon (4a-b) constituée au moins partiellement d'un matériau substantiellement transparent,
mise à disposition d'au moins un objet ou d'au moins une couche (9),
disposition de l'au moins objet ou de l'au moins une couche (9) dans le capuchon (3a-b), d'un côté intérieur de la coquille de capuchon (4a-b) conçu pour être tourné vers l'utilisateur pendant l'utilisation du capuchon (3a-b), ledit objet ou ladite couche (9) étant visible à travers la coquille de capuchon (4a-b),

8. Méthode selon la revendication 7, dans laquelle l'étape de mise à déposition d'une coquille de capuchon (4a-b) comprend au moins les étapes suivantes :
mise à disposition d'un moule (20) apte à être ouvert et fermé, définissant une cavité (21a-b) à l'état fermé,
apport d'au moins un matériau polymère substantiellement transparent, dans un état apte à être moulé, dans la cavité (21a-b) définie par le moule (20), pour produire une coquille de capuchon (4a-b), et
retrait de la coquille de capuchon (4a-b) produite dans le moule, hors du moule (20).

9. Méthode selon la revendication 8. comprenant en outre l'étape d'apport d'un objet (9) dans la cavité (21a-b), avant l'apport d'un matériau polymère dans la cavité (21a-b), lequel objet (9) est conçu pour être au moins partiellement moulé dans la coquille de capuchon (4a-b).

10. Méthode selon la revendication 8, comprenant en outre l'étape d'apport d'un objet ou d'une couche (9) dans la coquille de capuchon (4a-b), d'un côté de la coquille de capuchon (4a-b), après le retrait hors du moule (20) de la coquille de capuchon (4a-b) produite dans le moule (20), lequel côté est adapté pour être tourné vers l'utilisateur, et ledit objet ou laquelle couche (9) étant visible à travers la coquille de capuchon (4a-b).
